# EUROPÄISCHE PATENTSCHRIFT

(11) **EP 1 943 210 B1**
(45) Veröffentlichungstag und Bekanntmachung des Hinweises auf die Patenterteilung: **18.01.2012**
(21) Anmeldenummer: 06819107.1
(22) Anmeldetag: 20.10.2006
(51) Int. Cl.: C07C 209/60, C07C 211/03, C07C 211/07

(54) **VERFAHREN ZUR HERSTELLUNG VON ALKYLAMINEN DURCH UMSETZUNG VON OLEFINEN MIT AMMONIAK**
METHOD FOR PRODUCING ALKYL AMINES BY REACTING OLEFINS WITH AMMONIA
PROCEDE POUR PRODUIRE DES ALKYLAMINES CONSISTANT A FAIRE REAGIR DES OLEFINES AVEC DE L'AMMONIAC

(30) Priorität: 25.10.2005 DE 102005051044
(43) Veröffentlichungstag der Anmeldung: 16.07.2008
(73) Patentinhaber: BASF SE, 67056 Ludwigshafen (DE)
(72) Erfinder: ZEHNER, Peter, 67273 Weisenheim am Berg (DE); SIGL, Marcus, 68167 Mannheim (DE); BÖHLING, Ralf, 64653 Lorsch (DE)
(86) Internationale Anmeldenummer: PCT/EP2006/067602
(87) Internationale Veröffentlichungsnummer: WO 2007/048753

(56) Entgegenhaltungen:
- EP-A1- 0 721 934
- EP-A2- 0 822 179
- WO-A-01/85667

## Beschreibung

Die Erfindung betrifft ein Verfahren zur Herstellung von Alkylaminen durch Umsetzung von Olefinen mit Ammoniak unter hydroaminierenden Bedingungen an einem calcinierten zeolithischen Katalysator in einer adiabat betriebenen Reaktoreinheit.

Die Addition von Ammoniak an Olefinen ist exotherm; für die Verschiebung des Gleichgewichtes in Richtung des Wertproduktes Alkylamin sind daher niedrige Temperaturen vorteilhaft. Bei tiefen Temperaturen sind jedoch die Reaktionsgeschwindigkeiten gering. Daher wurde nach Katalysatoren gesucht, die auch bei relativ niedrigeren Temperaturen eine ausreichende Reaktionsgeschwindigkeit gewährleisten. Da für die Gleichgewichtslage neben niedrigen Temperaturen hohe Drücke vorteilhaft sind, wird in der Regel bei Überdruck, häufig im Bereich von 40 bis 500 bar, bevorzugt zwischen 150 und 300 bar, gearbeitet. Die Lage des Temperaturoptimums für Umsatz und Selektivität ist von der Natur des Olefins und des eingesetzten Katalysators abhängig und liegt meist im Bereich von 230 bis 320°C. Selbst beim Temperaturoptimum und unter erhöhtem Druck sind jedoch nur Umsätze zwischen 5 und 20 %, häufig zwischen 10 und 15 % beim einmaligen Durchgang durch den Reaktor erreichbar.

Verfahren zur Herstellung von Alkylaminen durch Umsetzung von Olefinen mit Ammoniak an calcinierten zeolithischen Katalysatoren sind beispielsweise in EP-A 132 736, EP-A 752 409, EP-A 822 179 oder US 6,809,222 beschrieben. EP-A-721934 offenbart die direkte kühlung des Reaktionsgemisches der Alkylaminsynthese mit Edukten bzw. mit Reaktionsgemisch, das aus dem Reaktor abgezogen, abgekühlt und demselben ernent zugeführt wird.

Es war demgegenüber Aufgabe der Erfindung, ein Verfahren zur Verfügung zu stellen, wonach sich die Ausbeute an Olefin in technisch einfacher Weise steigern lässt. Insbesondere sollten bestehende Hochdruckreaktoren in einfacher Weise nachgerüstet werden können, ohne dass das innere Apparatevolumen, durch das das Reaktionsgemisch strömt, nennenswert, das heißt um bis zu maximal 10 % verringert wird, und insbesondere ohne dass Einbauten für ein externes Kühlmittel erforderlich wären.

Die Lösung geht aus von einem Verfahren zur Herstellung von Alkylaminen durch Umsetzung von Olefinen mit Ammoniak unter hydroaminierenden Bedingungen an einem calcinierten zeolithischen Katalysator in einer adiabat betriebenen Reaktoreinheit.

Die Erfindung ist dadurch gekennzeichnet, dass das Reaktionsgemisch, enthaltend das Ausgangsolefin, Ammoniak und das entsprechende Alkylamin an einer oder mehreren

Stellen abgezogen und in indirekten thermischen Kontakt mit dem Reaktionsgemisch an einer oder mehreren Stellen in der Reaktoreinheit mit jeweils niedrigerer Konzentration an Alkylamin gegenüber der Stelle oder den Stellen von der oder von den das Reaktionsgemisch abgezogen wurde, gebracht wird.

Es wurde gefunden, dass es möglich ist, den Umsatz an Olefin in einem Verfahren zur Herstellung von Alkylaminen mit adiabater Reaktionsführung zu steigern, indem das Reaktionsgemisch von einer oder mehreren geeigneten Stellen der Reaktionseinheit genutzt wird, um durch indirekten Wärmetausch das Reaktionsgasgemisch an einer oder mehreren geeigneten Stellen zu kühlen, wobei stets ein Reaktionsgemisch mit niedrigerer Konzentration an Alkylamin genutzt wird, um durch indirekten Wärmetausch ein Reaktionsgemisch mit höherer Konzentration an Alkylamin zu kühlen.

Bei dieser speziellen Verfahrensführung entspricht zwar die Temperaturdifferenz zwischen Feedstrom und dem abgezogenen Produktgemisch der durch die Reaktionswärme hervorgerufenen adiabaten Temperaturerhöhung, die Reaktoraustrittstemperatur ist jedoch deutlich geringer als die maximale Reaktionstemperatur.

In einer besonders vorteilhaften Verfahrensvariante wird das Reaktionsgemisch, enthaltend das Ausgangsolefin, Ammoniak und das entsprechende Alkylamin in der Mitte des Strömungsweges desselben durch die Reaktoreinheit umgelenkt und in indirektem thermischem Kontakt mit dem bis zur Mitte des Strömungsweges im Reaktor geleiteten Reaktionsgemisch im Gegenstrom geführt. Bei dieser Verfahrensführung wird die höchste Temperatur im Reaktor nicht am Austritt des Reaktionsgemisches, sondern in der Mitte des Strömungsweges desselben durch den Reaktor erreicht.

Das aus einer ersten Reaktionseinheit auftretende Reaktionsgemisch kann vorteilhaft in eine oder auch mehrere weitere Reaktionseinheiten eingeleitet werden, die analog zur ersten Reaktionseinheit ausgebildet sind. Die einzelnen hintereinander geschalteten Reaktoreinheiten können in einer einzigen drucktragenden Hülle angeordnet sein.

Dadurch, dass zwei oder mehrere Reaktoreinheiten vorgesehen werden, kann die einzelne Reaktoreinheit mit geringerer Länge und entsprechend niedrigerem Druckverlust ausgelegt werden.

Für die Umsetzung des Olefins mit Ammoniak geht man zunächst in bekannter Weise vor, das heißt man mischt Ammoniak mit Olefin in einem molaren Verhältnis von 1 : 1 bis 10 :1, bevorzugt von 1 : 1 bis 5 : 1, und führt die Reaktion in einem Festbettreaktor oder einem Wirbelschichtreaktor bei einem Druck von 40 bis 500 bar, bevorzugt von 150 bis 300 bar und einer Temperatur von 80 bis 400°C, bevorzugt von 230 bis 320°C, in der Gasphase oder im überkritischen Zustand durch.

Eine Ausführungsform des Verfahrens besteht darin, dass man Ammoniak zusammen mit dem Olefin in einem molaren Verhältnis von 1 : 1 bis 5 : 1 gemischt einem Festbettreaktor, der den calcinierten zeolitischen Katalysator enthält, zuführt, und bei einem Druck von 40 bis 500 bar, bevorzugt von 150 bis 310 bar, insbesondere von 200 bis 280 bar, und einer Temperatur von 200 bis 350°C, bevorzugt von 230 bis 320°C, in einphasigem Zustand umsetzt.

Als Olefine können insbesondere lineare oder verzweigte aliphatische C₃- bis C₅-Olefine eingesetzt werden. Bevorzugte olefinische Edukte sind Buten und insbesondere Isobuten. Entsprechend wird als besonders bevorzugtes Hydroaminierungsprodukt, ausgehend von Ammoniak und Isobuten, das tert.-Butylamin erhalten.

Im erfindungsgemäßen Verfahren werden calcinierte zeolithische Katalysatoren eingesetzt. Dies bedeutet, dass die Aktivmasse der Katalysatoren aus Zeolithen aufgebaut ist. Üblicherweise enthalten zeolithische Katalysatoren noch Bindemittel, die zur Herstellung der Katalysatorformkörper erforderlich sind. Bei der Herstellung der Katalysatorformkörper aus entsprechenden Formmassen wird üblicherweise nach einer Trocknung noch calciniert.

Hierbei wird in der Regel eine Temperatur von mehr als 400°C benötigt, damit das Bindermaterial aushärtet. Die Maximaltemperatur ist beschränkt durch die Stabilität des Zeolithen, der bei Temperaturen von mehr als 550°C seine Kristallinität verliert. Die Calcinierung wird großtechnisch im Drehrohr bei einer Temperatur im Bereich von 400 bis 550°C, und einer Verweilzeit von 1 bis 4 Stunden durchgeführt. Im Labor wird üblicherweise in einem Ofen bei einer Temperatur von 480 bis 520°C und einem Zeitraum von 2 bis 16 Stunden gearbeitet.

Geeignete Katalysatoren für die Hydroaminierung von Olefinen mit Ammoniak und/oder einem primären Amin sind Zeolithe; insbesondere Faujasite wie X-, Y- und USY-Zeolith, Erionit, Chabazit, Mordenit, Offretit, Clinoptiolith, Pentasile wie ZSM-5 und ZBM-10, ZSM-11, ZSM-12, MCM-22, MCM-41, MCM-48, MCM-49, MCM-56, EMT, SSZ-26, SSZ-33, SSZ-37, CIT-1, PSH-3, NU-85, Beta sowie die borhaltigen Formen, wie zum Beispiel ZBM-11, H-Bor-ZSM-5, H-Bor-Beta, H-Bor-ZSM-11 sowie die gallium- oder titanhaltigen Formen. Sie zeichnen sich durch eine hohe Anzahl an katalytisch aktiven Zentren aus, kombiniert mit einer großen Oberfläche.

Die beschriebenen Zeolithe unterscheiden sich im Typ und in der Art der Nachbehandlung nach ihrer Herstellung (zum Beispiel thermische Behandlung, Dealuminierung, Säurebehandlung, Metallioneneintausch, etc.).

Beispiele für geeignete Zeolithe finden sich in US-A 4,375,002, US-A 4,536,602, EP-A 305 564, EP-A 101 921 und DE-A 42 06 992.

Auch die aus EP-A 133 938, EP-A 431 451 und EP-A 132 736 bekannten Zeolithe, bei denen es sich um Bor-, Gallium-, Alumino- und Eisensilikatzeolithe handelt, die gegebenenfalls wie beschrieben mit Alkali-, Erdalkali- und Übergangsmetallen dotiert werden können, sind geeignet.

Weiterhin sind zum Beispiel auch die aus CA-A 2 092 964 bekannten Beta-Zeolithe, die als kristalline Aluminosilikate bestimmter Zusammensetzung mit einer Porengröße von mehr als 5 Å definiert sind, geeignet.

Bevorzugt werden metall- oder halogenmodifizierte Beta-Zeolithe eingesetzt, wie zum Beispiel in DE-A 195 30 177 beschrieben.

Insbesondere geeignet sind auch Zeolith-Katalysatoren des Pentasil-Typs mit einem SiO₂/Al₂O₃-Molverhältnis von größer/gleich 10, wie sie in EP-A 132 736 offenbart sind.

Unter die Aluminiumphosphate und Silico-Alumophosphate fallen die kristallinen Systeme mit Zeolithstrukturen oder zeolithähnlichen Strukturen wie zum Beispiel SAPO-37, AlPO₄-5, SAPO-5, wie in DE-A 196 01 409 beschrieben, aber auch amorphe Systeme, wie sie zum Beispiel in DE-A 44 31 093 beschrieben sind. Sie besitzen allgemein die Formel AbO₃*P₂O₅*xSiO₂.

Die Katalysatoren können in Form von Pulver oder bevorzugt als Formkörper wie Kugeln, Stränge, Tabletten oder Splitt, eingesetzt werden. Für die Verformung können 10 bis 60 Gew.-% (bezogen auf die zu verformende Masse) Bindemittel zugesetzt werden. Als Bindemittel eignen sich verschiedene Aluminiumoxide, bevorzugt Boehmit, amorphe Alumosilikate mit einem molaren SiO₂/Al₂O₃-Verhältnis von 25:75 bis 95:5, Siliciumdioxid, bevorzugt hochdisperses SiO₂ wie zum Beispiel Silikasole, Gemische aus hochdispersem SiO₂ und hochdispersem Al₂O₃, hochdisperses TiO₂ sowie Tone.

Nach der Verformung werden die Extrudate oder Presslinge zweckmäßig bei 80 bis 150°C für 1 bis 16 Stunden getrocknet und bei 300 bis 500°C für 1 bis 16 Stunden calciniert, wobei die Calcinierung wie die Aktivierung auch direkt im Hydroaminierungsreaktor erfolgen kann.

In der Regel werden die Katalysatoren in der H-Form eingesetzt. Zur Erhöhung der Selektivität, der Standzeit und der Anzahl der möglichen Katalysatorregenerierungen kann man jedoch zudem verschiedene Modifizierungen an den Katalysatoren vornehmen.

Eine Modifizierung der Katalysatoren besteht darin, das man die unverformten Katalysatoren mit Alkalimetallen wie Na und K, Erdalkalimetallen wie Ca und Mg, Übergangsmetallen wie Mn, Fe, Mo, Cu, Zn und Cr, Edelmetallen und/oder seltenen Erdmetallen wie La, Ce und Y dotiert.

Eine vorteilhafte Katalysatorausführungsform besteht darin, dass man die verformten und calzinierten Katalysatoren in einem Strömungsrohr vorlegt und bei 20 bis 100°C zum Beispiel ein Halogenid, ein Acetat, ein Oxalat, ein Citrat oder ein Nitrat der oben beschriebenen Metalle in gelöster Form darüberleitet. Ein derartiger Ionenaustausch kann zum Beispiel an der Wasserstoff-, Ammonium- und Alkaliform der Katalysatoren vorgenommen werden.

Eine andere Möglichkeit der Metallaufbringung auf die Katalysatoren besteht darin, dass man das zeolithische Material zum Beispiel mit einem Halogenid, Acetat, Oxalat, Citrat, Nitrat oder Oxid der oben beschriebenen Metalle in wässriger oder alkoholischer Lösung imprägniert.

Sowohl an einen Ionenaustausch als auch an eine Imprägnierung kann man eine Trocknung, wahlweise eine abermalige Calcination anschließen. Bei den metalldotierten Katalysatoren kann eine Nachbehandlung mit Wasserstoff und/oder mit Wasserdampf günstig sein.

Eine weitere Möglichkeit der Modifizierung des Katalysators besteht darin, dass man das heterogenkatalytische Material - verformt oder unverformt - einer Behandlung mit Säuren, wie Salzsäure (HCl), Flusssäure (HF), Phosphorsäure (H₃PO₄), Schwefelsäure (H₂SO₄), Oxalsäure (HO₂C-CO₂H) oder deren Gemischen unterwirft.

Eine besondere Ausführungsform besteht darin, dass man das Katalysatorpulver vor seiner Verformung mit Flusssäure (0,001 bis 2 molar, bevorzugt 0,05 bis 0,5 molar) 1 bis 3 Stunden unter Rückfluss behandelt. Nach Abfiltrieren und Auswaschen wird in der Regel bei 100 bis 160°C getrocknet und bei 400 bis 550°C calciniert.

Eine weitere besondere Ausführungsform liegt in einer HCl-Behandlung der Heterogenkatalysatoren nach ihrer Verformung mit Bindemittel. Hierbei wird der Heterogenkatalysator in der Regel 1 bis 3 Stunden bei Temperaturen zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung des Katalysators ist der Austausch mit Ammoniumsalzen, zum Beispiel mit NH₄Cl, oder mit Mono-, Di- oder Polyaminen. Hierbei wird der mit Bindemittel verformte Heterogenkatalysator in der Regel bei 60 bis 80°C mit 10 bis 25%iger, bevorzugt ca. 20%iger NH₄Cl-Lösung 2 Stunden kontinuierlich in gewichtsmäßiger Heterogenkatalysator/Ammoniumchlorid-Lösung von 1:15 ausgetauscht und danach bei 100 bis 120°C getrocknet.

Eine weitere Modifikation, die an aluminiumhaltigen Katalysatoren vorgenommen werden kann, ist eine Dealuminierung, bei der ein Teil der Aluminiumatome durch Silicium ersetzt wird oder die Katalysatoren durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503.

Die Katalysatoren kann man als Stränge mit Durchmessern von zum Beispiel 1 bis 4 mm oder als Tabletten mit zum Beispiel 3 bis 5 mm Durchmesser für die Hydroaminierung der Olefine einsetzen.

Die Reaktoreinheit ist in der Regel ein Reaktor, vorteilhaft ein Schachtapparat, der mit Einbauten versehen ist. Diese Einbauten sind derart ausgebildet, dass kälteres und wärmeres Reaktionsgemisch in indirektem thermischem Kontakt im Gegenstrom aneinander vorbeigeleitet werden.

Die Einbauten können beispielsweise als Rohrbündel oder als Trennbleche im Schachtapparat ausgebildet sein.

Besonders vorteilhaft ist es, die Einbauten in der Weise auszulegen, dass der Wärmetransportweg für das Reaktionsgemisch zwischen 3 und 15 mm, insbesondere zwischen 5 und 10 mm beträgt.

Dabei entspricht der Wärmetransportweg in der konstruktiven Variante eines Rohrbündels dem Rohrinnendurchmesser und in der Variante mit Trennblechen dem Abstand zweier aufeinander folgender Trennbleche.

Die Erfindung wird im Folgenden durch ein Ausführungsbeispiel näher erläutert.

In einer Simulation wurde der maximal erreichbare Umsatz an Isobuten in einem Verfahren zur Herstellung von tert.-Butylaminen aus Isobuten und Ammoniak an einem zeolithischen Katalysator bei einem Reaktionsdruck von 280 bar und adiabaten Reaktionsbedingungen, ohne Kühlung des Reaktionsgemisches (zum Vergleich) und mit Kühlung des Reaktionsgemisches durch Umlenkung des Reaktionsgemisches in der Mitte des Strömungsweges desselben durch den Reaktor und indirekten Wärmetausch mit dem auf der ersten Hälfte des Strömungsweges durch den Reaktor zirkulierenden Reaktionsmedium (entsprechend der Erfindung) verglichen.

Die Einsatztemperaturen für den Eduktstrom waren jeweils umsatzoptimiert, das heißt es wurden jeweils die Einsatztemperaturen gewählt, bei denen der maximal erreichbare Umsatz erzielt wurde, und zwar eine Eintrittstemperatur 253°C für das Verfahren nach dem Vergleich bzw. eine Eintrittstempertur von 245°C für das erfindungsgemäße Verfahren. Im Verfahren nach dem Vergleich wurde ein Umsatz an Isobuten von 15,8 % erreicht, gegenüber einem Umsatz von 16,8 % für das erfindungsgemäße Verfahren.

## Patentansprüche

1. Verfahren zur Herstellung von Alkylaminen durch Umsetzung von Olefinen mit Ammoniak unter hydroaminierenden Bedingungen an einem calcinierten zeolithischen Katalysator in einer adiabat betriebenen Reaktoreinheit, **dadurch gekennzeichnet, dass** das Reaktionsgemisch, enthaltend das Ausgangsolefin, Ammoniak und das entsprechende Alkylamin an einer oder mehreren Stellen abgezogen und in indirekten thermischen Kontakt mit dem Reaktionsgemisch an einer oder mehreren Stellen in der Reaktoreinheit mit jeweils niedrigerer Konzentration an Alkylamin gegenüber der Stelle, von der das Reaktionsgemisch abgezogen wurde, gebracht wird.

2. Verfahren nach Anspruch 1 zur Herstellung von Alkylaminen durch Umsetzung von Olefinen mit Ammoniak unter hydroaminierenden Bedingungen an einem calcinierten zeolithischen Katalysator in einer adiabat betriebenen Reaktoreinheit, **dadurch gekennzeichnet, dass** das Reaktionsgemisch, enthaltend das Ausgangsolefin, Ammoniak und das entsprechende Alkylamin in der Mitte der Strömungswege desselben durch die Reaktoreinheit umgelenkt und in indirekten thermischen Kontakt mit dem bis zur Mitte der Strömungsweges in der Rektoreinheit geleiteten Reaktionsgemisch im Gegenstrom geführt wird.

3. Verfahren nach Anspruch 1 oder 2, **dadurch gekennzeichnet, dass** mehrere Reaktoreinheiten hintereinander geschaltet sind.

4. Verfahren nach einem der Ansprüche 1 bis 3, **dadurch gekennzeichnet, dass** die Olefine aus C₃- bis C₅-Olefinen ausgewählt sind.

5. Verfahren nach Anspruch 4, **dadurch gekennzeichnet, dass** das Olefin Iso-Buten ist.

6. Verfahren nach einem der Ansprüche 1 bis 5, **dadurch gekennzeichnet, dass** die Reaktoreinheit ein Schachtapparat mit Einbauten ist.

7. Verfahren nach Anspruch 6, **dadurch gekennzeichnet, dass** die Einbauten ein Rohrbündel oder Trennbleche sind.

8. Verfahren nach einem der Ansprüche 6 oder 7, **dadurch gekennzeichnet, dass** der Wärmetransportweg in den Einbauten 3 bis 15 mm beträgt.

9. Verfahren nach Anspruch 8, **dadurch gekennzeichnet, dass** der Wärmetransportweg 5 bis 10 mm beträgt.

10. Verfahren nach Anspruch 8 oder 9, **dadurch gekennzeichnet, dass** der Wärmetransportweg dem Innendurchmesser der Rohre des Rohrbündels oder dem Abstand zweier aufeinander folgender Trennbleche entspricht.

## Claims

1. A process for preparing alkylamines by reacting olefins with ammonia under hydroaminating conditions over a calcined zeolitic catalyst in an adiabatically operated reactor unit, wherein the reaction mixture comprising the starting olefin, ammonia and the corresponding alkylamine is taken off at one or more points and brought into indirect thermal contact with the reaction mixture at one or more points in the reactor unit having in each case a lower concentration of alkylamine compared to the point from which the reaction mixture was taken off.

2. The process according to claim 1 for preparing alkylamines by reacting olefins with ammonia under hydroaminating conditions over a calcined zeolitic catalyst in an adiabatically operated reactor unit, wherein the reaction mixture comprising the starting olefin, ammonia and the corresponding alkylamine is diverted in the middle of its flow path through the reactor unit and put into indirect countercurrent thermal contact with the reaction mixture conveyed up to the middle of the flow path in the reactor unit.

3. The process according to claim 1 or 2, wherein a plurality of reactor units are connected in series.

4. The process according to any of claims 1 to 3, wherein the olefins are selected from among C₃-C₅-olefins.

5. The process according to claim 4, wherein the olefin is isobutene.

6. The process according to any of claims 1 to 5, wherein the reactor unit is a shaft apparatus provided with internals.

7. The process according to claim 6, wherein the internals are separation plates or a bundle of tubes.

8. The process according to either claim 6 or 7, wherein the heat transport path in the internals is from 3 to 15 mm.

9. The process according to claim 8, wherein the heat transport path is from 5 to 10 mm.

10. The process according to claim 8 or 9, wherein the heat transport path corresponds to the internal diameter of the tubes of the bundle of tubes or to the distance between two successive separation plates.

## Revendications

1. Procédé pour la préparation d'alkylamines par transformation d'oléfines avec de l'ammoniac dans des conditions d'hydroamination sur un catalyseur zéolithique calciné dans une unité de réacteur exploitée de manière adiabatique, **caractérisé en ce que** le mélange réactionnel, contenant l'oléfine de départ, de l'ammoniac et l'alkylamine correspondante est soutiré en un ou différents sites et est amené en contact thermique indirect avec le mélange réactionnel en un ou plusieurs sites dans l'unité de réacteur avec des concentrations à chaque fois inférieures en alkylamine par rapport au site où le mélange réactionnel a été soutiré.

2. Procédé selon la revendication 1 pour la préparation d'alkylamines par transformation d'oléfines avec de l'ammoniac dans des conditions d'hydroamination sur un catalyseur zéolithique calciné dans une unité de réacteur exploitée de manière adiabatique, **caractérisé en ce que** le mélange réactionnel, contenant l'oléfine de départ, l'ammoniac et l'alkylamine correspondante est dévié au milieu de la voie d'écoulement de celui-ci dans l'unité de réacteur et est guidé à contre-courant, en contact thermique indirect, par rapport au mélange réactionnel guidé jusqu'au centre de la voie d'écoulement dans l'unité de réacteur.

3. Procédé selon la revendication 1 ou 2, **caractérisé en ce que** plusieurs unités de réacteur sont commutées les unes derrière les autres.

4. Procédé selon l'une quelconque des revendications 1 à 3, **caractérisé en ce que** les oléfines sont choisies parmi les C₃-C₅-cléfines.

5. Procédé selon la revendication 4, **caractérisé en ce que** l'oléfine est l'isobutène.

6. Procédé selon l'une quelconque des revendications 1 à 5, **caractérisé en ce que** l'unité de réacteur est un appareil à cuve avec des inserts.

7. Procédé selon la revendication 6, **caractérisé en ce que** les inserts sont un faisceau tubulaire ou des tôles de séparation.

8. Procédé selon l'une quelconque des revendications 6 ou 7, **caractérisé en ce que** la voie de transport thermique dans les inserts est de 3 à 15 mm.

9. Procédé selon la revendication 8, **caractérisé en ce que** la voie de transport thermique est de 5 à 10 mm.

10. Procédé selon la revendication 8 ou 9, **caractérisé en ce que** la voie de transport thermique correspond au diamètre interne des tubes du faisceau tubulaire ou à la distance de deux tôles de séparation consécutives.
